# EUROPEAN PATENT APPLICATION

(11) **EP 0 546 951 A1**
(43) Date of publication of application: **16.06.1993**
(21) Application number: 92403374.9
(22) Date of filing: 11.12.1992
(51) Int. Cl.: A61K 37/02

(54) **Combination of liposome encapsulated antineoplastic agents, such as doxorubicin with colony stimulating factors**

(30) Priority: 13.12.1991 US 807116; 09.12.1992 US 988164
(71) Applicant: THE LIPOSOME COMPANY, INC., Princeton, NJ 08540 (US)
(72) Inventor: Ostro, Marc J., Pennington, New Jersey 08534 (US)
(74) Representative: Warcoin, Jacques

(57) **Abstract**

A method of treating a neoplasm by alleviating myelosuppression as a dose limiting toxicity associated with the administration of a liposome encapsulated antineoplastic agent by administering a hematopoietic cell stimulating agent, such as a colony stimulating factor. A preferred antineoplastic agent is an antibiotic such as doxorubicin.

## Description

### RELATED COPENDING U.S. APPLICATION DATA

This application is a continuation-in-part of U.S. Patent Application Serial No. 07/807.116, filed December 13, 1991.

### FIELD OF THE INVENTION

This invention relates to methods of treating a neoplasm with liposome encapsulated antineoplastics and hematopoietic cell stimulating agents.

### BACKGROUND OF THE INVENTION

Antineoplastics agents are cytotoxic drugs that include plant alkaloids, antibiotics, antimetabolites, alkylating agents and miscellaneous agents such as amsacrine and mitoxanthrone.

A major drawback to the use of antineoplastic agents in the treatment of neoplasms has been their dose limiting toxicity. Hematological toxicities include bone marrow suppression with neutropenia, resulting in fevers and/or infections and anemia. General systemic toxicities often associated with antineoplastic agents include alopecia, stomatitis, gastrointestinal disturbances including nausea and vomiting, mucositis, dysphagia, diarrhea, tissue necrosis, phlebitis, fever and chills. Reversible hepatic dysfunction may also occur.

Myelosuppression associated with the administration of antineoplastic agents is usually dose related (O'Bryan et al., Cancer 1973; 32:1-8; Wheeler et al. Cancer Treat Rep 1982; 66:493-498; Bronchud et al., Br. J. Cancer 1989;60:121-125). In most cases it is reversible with the discontinuation of treatments, reduction in the frequency of treatments or dose reduction (Cortes et al., Cancer Treat Rep 1978;62:217-277; Morstyn et al. Lancet 1988;26:667-672).

Doxorubicin, an antibiotic, is useful as an antineoplastic drug produced by the fermentation products of Streptomyces peucetius caesius. Antibiotics such as doxorubicin have been utilized against many forms of cancer including solid tumors, breast, lung and ovarian cancer, sarcomas, lymphomas and leukemias (Schoenfeld et al., Cancer 1982; 5:2757-2762; O'Bryan et al., Cancer 1973; 32:1-8).

Dose escalation of antibiotics such as doxorubicin is often restricted by cardiac muscle damage. It is believed that doxorubicin cardiac toxicity is associated with peroxidation of cardiac lipids. Cardiomyopathy, the most common cardiac toxicity, is usually evident at cumulative doses of 550 mg/m². Various approaches to the prevention of myocardial damage have included the co-administration of antioxidants (Myers et al., Science 1977; 197:165-167; Doroshow et al. J Clin Invest 1981; 68:1053-1064), the use of a cardioprotectant (Speyer et al., N Engl J Med 1988; 319:745-752) or analogues complexing free doxorubicin with DNA (Guven et al., Cancer Chemother Pharmocol 1986; 17:153-156). None of these strategies have proven satisfactory for clinical use.

Liposomes are completely closed lipid bilayer membranes containing an entrapped aqueous volume. Liposomes may be unilamellar vesicles, possessing a single bilayer membrane, of multilamellar vesicles, onion-like structures characterized by multiple membrane bilayers, each separated from the next by an aqueous layer. The bilayer is composed of two lipid monolayers having a hydrophobic "tail" region and hydrophilic "head" region. The structure of the membrane bilayer is such that the hydrophobic (nonpolar) "tails" of the lipid monolayers orient toward the center of the bilayer while the hydrophilic "head" orient towards the aqueous phase.

In a liposome-drug delivery system, a bioactive agent such as a drug is entrapped in the liposome and then administered to the patient to be treated. For example, see Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179; Lenk et al., U.S. Patent No. 4,522,803; and Fountain et al. U.S. Patent No. 4,588,578.

Encapsulation of antineoplastic agents within liposomes has been shown to ameliorate myocardial and gastrointestinal damage when compared with similar doses of conventional doxorubicin (Rahman et al., Cancer Res. 1985, 45:796-803; Herman et al., Cancer Res. 1983, 43:5427-5432). Other potential benefits in using liposomes in antineoplastic therapy include a reduction in drug toxicity by the targeting the drug away from sites of toxicity; enhancing the potency of drugs via slow release of drugs from liposomes circulating in the bloodstream or localized in reticuloendothelial cells in the liver, spleen and bone marrow; reducing cardiotoxicity by the slow release of drug; and allowing concentration of drug at frequent sites of metastatic disease, such as marrow, liver or bone marrow.

Liposome encapsulated doxorubicin has been shown to reduce toxicities, such as mucositis, while maintaining or improving the efficacy of the drug, Rahman et al., Cancer Chemother Pharmacol. 1986;16:22-27; Bally et al., "Encapsulation of Antineoplastic Agents in Liposomes", PCT Application No. 85/01501. Publication No. WO 86/01102 published February 27, 1986, and Mayer et al. "Low Toxicity Formulations of Liposomal-Antineoplastic Agents" PCT Application No. 88/00646, PCT Publication No. WO88/06442, published September 7, 1988.

The pharmacokinetics of liposome encapsulated doxorubicin often differs from that of the free drug in that there is a higher plasma level and greater area under the curve and also by the slow conversion of liposome encapsulated doxorubicin to metabolites doxorubicinol and doxorubicinone (Rahman et al., J Clin Onocol, 1990; 8:1093-1100).

Colony stimulating factors are a class of glycoprotein hormones that effect the growth and differentiation of hematopoietic progenitor cells. These factors are produced by monocytes, fibroblasts and endothelial cells and regulate the production of neutrophils, among other blood components, within the bone marrow. Colony stimulating factors include: (i) granulocyte macrophage colony stimulating factor (GM-CSF) which promotes colonies made of both granulocytes and macrophages, (ii) granulocyte colony stimulating factor (G-CSF) which leads to the development of colonies consisting of granulocytes, (iii) and macrophage colony stimulating factor (M-CSF) which leads to the development of colonies consisting of macrophages. These colony stimulating factors have been purified, molecularly cloned and expressed as recombinant proteins (Seelentag et al., EMBO J 1987; 6:2261-2265).

Recombinant human granulocyte colony stimulating factor has been shown to effectively shorten the period of antineoplastic drug induced muelosuppression (Welte et al., J Exp Med 1987; 72:2074-2081; Morstyn et al., Lancet 1988;26:667-672).

Studies have shown that the administration of granulocyte colony stimulating factor to patients with non-myeloid malignancies resulted in a dose-dependent increase in circulating neutrophils. This increase was evident whether the agent was administered by intravenous injection twice a day, subcutaneously daily or by continuous subcutaneous infusion. It has also been found that with the discontinuation of therapy, neutrophil counts returned to baseline in most cases within a few days (Gabrilove et al., J Clin Invest 1988; 82:1454-1461; Morstyn et al., Lancet 1988;26:667-672; Bronchud et al., Br J Cancer 1987; 56:809-813).

Recently G-CSF has been shown to be safe and effective in accelerating the recovery of neutrophil counts following a variety of chemotherapy regimens (Gabrilove et al., N Engl J Med 1988; 318:1414-1422; Neidhart et al., J Clin Oncol 1989; 7:1985-1691; Bronchud et al., Br J Cancer 1989; 60: 121-128). Studies have shown that the incidence, severity and duration of severe neutropenia following chemotherapy were all significantly reduced.

One aspect of the present invention discloses a method of treating neoplasms which ameliorates myelosuppression as a dose limiting toxicity associated with liposomal encapsulated antineoplastic agents, for example antibiotics such as doxorubicin. This treatment requires the administration of hematopoietic cell stimulating agents, such as a colony stimulating factor, and liposome encapsulated antineoplastic agents, such as doxorubicin. This treatment allows for the escalation of dosages of the antineoplastic agent previously thought to be toxic.

### SUMMARY OF THE INVENTION

The present invention provides for methods of treating neoplasms comprising the administration of an antineoplastic effective amount of a liposome encapsulated antineoplastic agent and a hematopoietic cell stimulating effective amount of a hematopoietic cell stimulating agent.

The antineoplastic agents can be but are not limited to plant alkaloids, antibiotics, antimetabolites, alkylating agents and miscellaneous agents such as amsacrine and mitoxanthrone. Preferably the antineoplastic agent is an antibiotic, such as doxorubicin.

Hematopoietic cell stimulating agents include colony stimulating factors, interleukins, erythropoetin and platelet stimulating factors. Preferably the hematopoietic cell stimulating agent is a colony stimulating factor. The colony stimulating factors are preferably G-CSF, GM-CSF and M-CSF.

The liposome can comprise a chemical potential across its outer bilayer membrane and more preferably this chemical potential is a pH gradient.

Preferably the liposome is comprised of a phospholipid.

This invention also provides a method wherein the administration of the colony stimulating factor is alternated with the administration of the antineoplastic agent.

This invention also provides a method wherein the dosage of the liposome encapsulated antineoplastic agent is escalated. Preferably the antineoplastic agent is a antibiotic such as doxorubicin and the hematopoietic cell stimulating agent is a colony stimulating factor.

This invention also provides a method wherein the maximum tolerated dose associated with the administration of the liposome encapsulated antineoplastic agent is increased with the use of the hematopoietic cell stimulating agent so that myelosuppression is alleviated as a dose limiting toxicity.

The present invention additionally provides a method of alleviating myelosuppression as a dose limiting toxicity associated with the administration of a liposome encapsulated antineoplastic agent to patients with neoplasms by administering an escalating dose of a liposome encapsulated antineoplastic agent, such as antibiotics, for example doxorubicin, and a hematopoietic cell stimulating agent, preferably a colony stimulating factor.

### DETAILED DESCRIPTION OF THE INVENTION

As described above, the present invention relates to methods of treating neoplasms utilizing hematopoietic cell stimulating agents and liposome-encapsulated antineoplastics. Antineoplastic agents useful in the treatment of neoplasms are often associated with myelosuppression. It has now been discovered that associated myelosuppression as the dose limiting toxicity may be ameliorated with the administration of hematopoietic cell stimulating agents. Hematological dose limiting toxicity at heretofore maximum tolerated dosages is alleviated. Therefore, the maximum tolerated dosage commonly attributed to these antineoplastic agents is increased and a nonmyelosuppressive toxicity becomes dose limiting. Since the nonmyelosuppressive toxicity associated with liposome-encapsulated antineoplastic agents are generally reduced, the maximum tolerated dose of a liposome-encapsulated antineoplastic agent given along with a hematopoietic cell stimulating agent will be higher than the maximum tolerated dose obtainable with free antineoplastic agent alone, free antineoplastic agent plus hematopoietic cell stimulating agents, or liposomal antineoplastic agent without a hematopoietic cell stimulating agent.

Neoplasm is defined in the present invention as any abnormal growth or proliferation of tissue or cells in the body.

Treatment is defined in the present invention as any form of management and care for the purpose of combating, preventing or controlling a disease of disorder including but not limited to the administration of a liposome encapsulated antineoplastic agent and hematopoietic cell stimulating agent to ameliorate, prevent, i.e. prophylaxis, maintain and/or control the disease, disorder or complications arising therefrom.

The method of this invention can be used with any antineoplastic agent having an adverse hematopoietic effect and more specifically causing myelosuppression or adversely effecting bone marrow in the production of granulocytes and white blood cells. Examples of antineoplastic agents envisioned in the practice of the invention are plant alkaloids, for example vinblastine, vindesine, etoposide and teniposide; antibiotics, for example dactinomycin, doxorubicin, daunorubicin and mitomycin; antimetabolites, for example methotrexate, methotrexate with leucovorin, 5-fluorouracil, 6-mecaptopurine, 6-thioguanine, cytarabine, 5-azacytidine and hydroxyurea; alkylating agents, for example nitrogen mustards, mechlorethamine, cyclophosphamide, melphalan, uracil mustard and chlorambucil; busulfan, carmustine, lomustine, semustine, ifosfamide, mesna, thiotepa and carboplatin; and miscellaneous agents for example mitoxanthrone and amsacrine. The antineoplastic agents are preferably antibiotics and more preferably doxorubicin.

Hematopoietic cell stimulating agents, for example colony stimulating factors, interleukins, erythopoietin and platelet stimulating factors are defined in the present invention as any agents which promote the formation of blood cells. Examples of colony stimulating factors envisioned in the practice of this invention include but are not limited to granulocyte colony stimulating factor (G-CSF), granulocyte-macrophage colony stimulating factor (GM-CSF) and macrophage (M-CSF). Colony stimulating agent would not be recommended for treatment of myeloid malignancies due to the myeloid cell stimulating properties of these particular hermatopoietic cell stimulating agents.

Dose limiting toxicity is defined in the present invention as the dose at which hematological or nonhematological toxicities are life threatening.

Hematological toxicity is defined in the present invention as neutropenia, a substantially reduced neutrophil count, for example, granylocyte count of less than or equal to 900/ml; thrombocytopenia, for example, a platelet count of less than or equal to 49,000/ml; leukocytosis; or anemia.

Nonhematological toxicity is defined as any symptom or disorder associated with the treatment regimen which substantially effects the health or well being of the recipient. Examples of nonhematological toxicities include but are not limited to anaphylaxis, alopecia, stomatitis, ulceration, gastrointestinal disturbances including nausea and vomiting, mucositis, dysphagia, diarrhea, rash, tissue necrosis, phlebitis, renal or hepatic dysfunction, fever, chills or cardiac toxicity, such as an abnormal or changed electrocardiogram, disturbance in cardiac rhythm or a drop in the ejection fraction, generally considered significant if a reduction of about 15% or more, or cardiomyopathy.

Maximum tolerated dose is defined in the present invention as the dose at which dose limiting toxicity is exhibited.

The antineoplastic agent or agents are encapsulated in a liposome. The liposomes may be prepared by means known in the art. As to liposome preparation, the original liposome preparation of Bangham, et al. (J. Mol. Biol., 1965, 13:238-252) involves supending phospholipids in an organic solvent which is then evaporated to dryness leaving a phospholipid film on the reaction vessel. Next, an appropriate amount of aqueous phase is added, the mixture is allowed to "swell," and the resulting liposomes which consist of multilamellar vesicles (MLVs) are dispersed by mechanical means. This technique provides the basis for the development of the small sonicated unilamellar vesicles described by Papahadjopoulos et al. (Biochim. Biophys. Acta., 1968, 135:624-638), and large unilamellar vesicles.

Large unilamellar vesicles may be produced using an extrusion apparatus by a method described in Cullis et al., U.S. Patent No. 5,008,050 issued April 16, 1991 and PCT Publication No. WO 86/00238, published January 16, 1986, entitled "Extrusion Technique for Producing Unilamellar Vesicles" incorporated herein by reference. Vesicles made by this technique, called LUVETS, are extruded under pressure through a membrane filter. Vesicles may also be made by an extrusion technique through a 200nm filter, such vesicles are known as VET₂₀₀s. These vesicles may be exposed to at least one freeze and thaw cycle prior to the extrusion technique; this procedure is described in Mayer, et al., (Biochim. Biophys. Acta., 1985, 817:193-196), entitled "Solute Distributions and Trapping Efficiencies Observed in Freeze-Thawed Multilamellar Vesicles".

Other techniques that are used to prepare vesicles include those that form reverse-phase evaporation vesicles (REV), Papahadjopoulos et al., U.S. Patent No. 4,235,871. Another class of liposomes are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 5,030,453 to Lenk et al. issued July 9, 1991, and includes SPLV's in U.S. Patent No. 4558,579 to Fountain et al. and frozen and thawed multilamellar vesicles (FATMLV) wherein the vesicles are exposed to at least one freeze and thaw cycle; this procedure is described in Bally et al., U.S. Patent No. 4,975,282 issued December 4, 1990 and PCT Publication No. 87/00043, published January 15, 1987, entitled "Multilamellar Liposomes Having Improved Trapping Efficiencies" and incorporated herein by reference.

A variety of sterols and their water soluble derivatives have been used to form liposomes; see specifically Janoff et al., U.S Pat. No. 4,721,612 issued January 26, 1988, entitled "Steroidal Liposomes." Mayhew et al., PCT Publication No. WO 85/00968, published March 14, 1985, describing a method for reducing the toxicity of drugs by encapsulating them in liposomes comprising alpha-tocopherol and certain derivatives thereof. Also, a variety of tocopherols and their water soluble derivatives have been used to form liposomes, see Janoff et al., U.S. Patent No. 4,861,580 issued August 29, 1989, PCT Publication No. WO 87/02219, published April 23, 1987, entitled "Alpha Tocopherol-Based Vesicles."

The method for preparing the sterol vesicles involves adding to an aqueous buffer a salt form of an organic acid derivative of a sterol capable of forming closed bilayers in an amount sufficient to form completely closed bilayers which entrap an aqueous compartment. A suspension of multilamellar vesicles is formed by shaking the mixture. The formation of vesicles is facilitated if the aqueous buffer also contains the counterion of the salt in solution.

The application of energy to the suspension, e.g. sonication, or extrusion of the vesicles through a French press cell or through a porous filter of the appropriate pore size, will convert the multilamellar sterol vesicles to unilamellar vesicles.

The liposomes useful in the practice of the present invention can be multilamellar or unilamellar. Multilamellar liposomes may be made by any method known in the art and include SPLVs, freeze and thaw MLVs, REVs and MPVs.

Unilamellar liposomes may be formed by a freeze and thaw technique followed by an extrusion through polycarbonate filters. Liposomes of a predetermined size may also be formed by passing the a suspension of liposomes under pressure one or more times through an aluminum oxide porous film, Coe et al. "Liposome Extrusion Process "U.S. Patent Application Ser. No. 771,267, a continuation -in-part of U.S. Patent Application Ser. No. 593,200 filed October 5, 1990.

The liposomes may be dehydrated, thereby enabling storage for extended periods of time until use. Standard freeze-drying equipment or equivalent apparatus may be used to dehydrate the liposomes. Liposomes may be also be dehydrated simply by placing them under reduced pressure. Alternatively, the liposomes and their surrounding medium can be frozen in liquid nitrogen prior to dehydration. Dehydration with prior freezing may be performed in the presence of one or more protective sugars in the preparation, according to the process of Janoff et al., U.S. Patent No. 4,880,635 issued November 4, 1989 entitled "Dehydrated Liposomes". Alternatively multilamellar vesicles may be dehydrated with prior freezing without protective sugars. When the dehydrated liposomes are to be used, rehydration is accomplished by simply adding an aqueous solution, e.g., distilled water, to the liposomes and allowing them to rehydrate.

Lipids which can be used in the liposome formations of the present invention include synthetic or natural phospholipids and may include, but are not limited to, phosphatidylcholine (PC), phosphatidylethanolamine (PE), phosphatidylserine (PS), phosphatidylglycerol (PG), phosphatidic acid (PA), phosphatidylinositol (PI), sphingomyelin (SPM), cardiolipin, and the like, alone or in combination. The phospholipids can be synthetic or derived from natural sources such as egg or soy. Useful synthetic phospholipids are dymyristoylphosphatidylcholine (DMPC) and dimyristoylphosphatidylglycerol (DMPG). In other embodiments, distearylphosphatidylcholine (DSPC), dipalmitoylphosphatidylcholine (DPPC), dimyristoylphosphatidylcholine (DMPC), and diarachidonoylphosphatidylcholine (DAPC) may be used. The liposomes can also contain other steroid components such as coprostanol, cholestanol, or cholestane, polyethylene glycol derivatives of cholesterol (PEG-cholesterols). They may also contain organic acid derivatives of sterols such as cholesterol hemisuccinate (CHS), and the like. Organic acid derivatives of tocopherols may also be used as liposome-forming ingredients, such as alpha-tocopherol hemisuccinate (THS).Both CHS- and THS-containing liposomes and their tris salt forms may generally be prepared by any method known in the art for preparing liposomes containing these sterols. In particular, see the procedures of Janoff, et al., U.S. Pat. No. 4,721,612 issued January 26, 1988, entitled "Steroidal Liposomes." and Janoff, et al., U.S. Patents No. 4,861,580, issued August 29, 1989, and U.S. Patent No. 5,041,278 issued August 20, 1991, and PCT Publication No. No 87/02219, published April 23, 1987, entitled "Alpha Tocopherol-Based Vehicles". The liposomes may also contain glycolipids. Preferably the liposomes are comprised of phopholipids.

In the present invention the encapsulation of antineoplastic agent in liposomes may be accomplished by any of the methods known in the art, but preferably they are formed according to the procedures disclosed in Bally et al., "Encapsulation of Antineoplastic Agents in Liposomes". PCT application No. 85/01501, publication number WO 86/01102 published February 27, 1986 and Mayer et al. "High Drug:Lipid Formulations of Liposomal Encapsulated Antineoplastic Agent" PCT application number 88/00646, PCT publication number WO88/06442, published September 7, 1988 and Madden et al., "Accumulation of Drugs into Liposomes by a Proton Gradient" PCT publication WO 90/14105 published November 19, 1990, PCT application WO 90/02736, filed May 15, 1990. These techniques allow the loading of liposomes with ionizable pharmaceutical agents to achieve interior concentrations considerably greater than otherwise expected from the drugs solubility in aqueous solution at neutral pH and/or concentrations greater than can be obtained by passive entrapment techniques. Using this technique, a transmembrane ion, such as a pH gradient is created between the internal and external membranes of the liposomes and the pharmaceutical agent is loaded into the liposomes by means of the ion (pH) gradient, which drives the uptake. The transmembrane gradient is generated by creating a concentration gradient for one or more charged species, for example Na⁺, Cl⁻, K⁺, Li⁻, OH⁻ and preferably H⁺, across the liposome membranes, such that the ion gradient drives the uptake of the ionizable pharmaceutical agents across the membranes.

Bally et al. is directed to improved methods for encapsulating antineoplastic agents in liposomes and methods for reducing the rate of release of antineoplastic agents from liposomes. Liposomes are loaded with ionizable antineoplastic agents wherein a transmembrane potential is created across the walls of the liposomes and the antineoplastic agent is loaded into the liposomes by means of the transmembrane potential. The transmembrane potential is generated by creating a concentration gradient for one or more charged species (for ex. Na⁺, K⁺ and/or H⁺) across liposome membranes. The concentration gradient is created by producing liposomes having different internal and external media, i.e. concentrations of the one or more charged species.

Bally et. al. teaches liposomes which encapsulate a medium having a concentration of one or more charged species. This medium is also the external medium of the liposome. The original external medium is replaced by a one having a different concentration of the one or more charged species. The transmembrane potential is formed spontaneously or with the aid of a permeability enhancing agent , such as an ionophore, added to the bathing medium. Next the antineoplastic agent is added to the external medium and the transmembrane potential loads the agent into the liposome. The rate of release of the agent can be controlled by orientation of the transmembrane potential or by adjusting the concentrations of the inside and outside of the liposomes of a charged species such as Na⁺, K⁺ and/or H⁺. Further, the loaded or unloaded liposomes can be dehydrated for extended storage, etc. and rehydrated at the time of use without losing a substantial portion of their internal contents. If the unloaded liposomes are dehydrated the concentration gradient can be created either before hydration of after rehydration.

Mayer et al. is directed to a liposome composition of an antineoplastic agent and a lipid wherein the liposomes have a transmembrane ion gradient. The liposomes contain a release-inhibition buffer such as citric acid/sodium carbonate. The compositions are prepared by first forming the liposomes in an acidic buffer aqueous environment, alkalinizing the external medium of the liposomes with a base and adding the resultant liposomes to a antineoplastic agent. After a brief period of incubation of the liposome-drug suspension, the antineoplastic agent is entrapped in the liposome in response to the pH gradient. The drug to lipid weight ratios used can be as high as about 3:1.

Madden et al. discloses compositions having a pH gradient which exhibit markedly increased accumulation of pharmaceutical agents above that expected from the Henderson-Hasselbach relationship by formulating the liposomes utilizing a first internal aqueous buffer and a second external aqueous buffer wherein the concentration of the pharmaceutical agent exceeds its solubility product in the internal buffer following uptake. The pharmaceutical agent exhibits a solubility within the liposome which is less than the final concentration of agent within the liposomes. The solubility of the pharmaceutical agent is preferably less than about 20nm and the internal buffer solution has a buffer strength of at least about 50m Mol.

Conveniently, liposomal encapsulated antineoplastic agents for use in the practice of this invention may be prepared from a 3-vial kit, Mayer et al. "High Drug:Lipid Formulations of Liposomal Encapsulated Antineoplastic Agent" PCT Application No. 88/00646, PCT Publication No. WO88/06442, published September 7, 1988.

The mode of administration of the liposomes containing the antineoplastic agents of the present invention may determine the sites and cells in the organism to which the compound may be delivered. The liposomes of the present invention may be administered alone but will generally be administered in admixture with a pharmaceutical carrier selected with regard to the intended route of administration and standard pharmaceutical pratice. For parenteral administration or injection via intravenous, intraperitoneal, intramuscular, subcutaneous, or intra-mammary route, sterile solutions of the liposome composition are prepared. For intravenous use, the total concentration of solutes should be controlled to render the preparation isotonic. Other uses, depending upon the particular properties of the preparation, may be envisioned by those in the art.

The administration of a hematopoietic cell stimulating agent may be prior to, simultaneously with or post administration of the liposome encapsulated antineoplastic agent. It may be administered by any method known in the art including but not limited to subcutaneously, intravenously daily or by continuous subcutaneous infusion. It may be administered with a pharmaceutically acceptable carrier or diluent.

For administration to humans in the treatment of neoplasms, the prescribing physician will ultimately determine the appropriate dosage of the antineoplastic agent and hematopoietic cell stimulating agent for a given human subject, and this can be expected to vary according to the age, weight, and response of the individual as well as the pharmacokinetics of the agent used. Also the nature and severity of the patient's disease state or pharmacological condition will influence the dosage regimen.

The following example is given for purposes of illustration only and are not to be viewed as a limitation of the scope of the invention.

### EXAMPLE 1

### PREPARATION OF LIPOSOME ENCAPSULATED DOXORUBICIN(20 MG)

Liposome encapsulated doxorubicin is prepared using the 3-vial kit described in Mayer et al. "High Drug:Lipid Formulations of Liposomal Encapsulated Antineoplastic Agent" PCT Application No. 88/00646, PCT Publication No. WO88/06442, published September 7, 1988) A first vial contains the vehicle, egg phosphatidylcholine (71.3 mg/ml)/cholesterol (28.7 mg/ml) liposomes, less than 1.0 micron in size, in a concentration of 100 mg of total lipid per ml of citric acid buffer (300 mmol). The second vial contains the alkalinizing agent, a sodium carbonate injection 53 mg/ml (90.5 M). The third vial contains the active agent , doxorubicin HCL (20 mg), lactose an methylparaben (1:5.0:1 wt. ratio) as a lyophilized powder conveniently available from Adria Laboratories.

Allow the first and second vials to come to room temperature. Turn water bath on. Allow the water to equilibrate at a temperature of 58°C. Withdraw 8.8 ml of Sodium Chloride injection 0.9% and inject into the 20 mg doxorubicin HCL vial (vial three). Shake well. Withdraw 2.7 ml of alkalinizing agent and inject into a vented vial of the vehicle (vial one). Remove the vent needle and shake well. Using a 3 ml syringe withdraw 1.2 ml of vehicle/alkalinizing agent and inject into the vented vial of 20 mg doxorubicin HCl. Heat the vial in a water bath within a temperature range of 55-66°C for one minute. Shake vigorously for 10 seconds. Repeat this step two more times. Then heat the vial in a water bath at a temperature within a range of 55-66°C for an additional seven minutes and shake vigorously. Use the doxorubicin HCl liposome injection within 8 hours. Refrigerate the vehicle and alkalinizing vials within a temperature range of 2-8°C until the time of use. The active agents is held at controlled room temperature range of 15-30°C.

### EXAMPLE 2

### PREPARATION OF LIPOSOME ENCAPSULATED DOXORUBICIN (50 MG)

A preparation of 50 mg of doxorubicin encapsulated in a liposome may be prepared according to Example 1 using a 50 mg vial of doxorubicin HCl (vial three).

Allow the first and second vials to come to room temperature. Turn water bath on. Allow the water to equilibrate at a temperature of 58°C. Withdraw 22.0 ml of Sodium Chloride injection 0.9% and inject into the 50 mg doxorubicin HCl vial (vial three). Shake well. Withdraw 2.7 ml of alkalinizing agent and inject into a vented vial of the vehicle (vial one). Remove the vent needle and shake well. Using a 3 ml syringe withdraw 3.0 ml of vehicle/alkalinizing agent and inject into the vented vial of 50 mg doxorubicin HCl. Heat the vial in a water bath within a temperature range of 55-66°C for one minute. Shake vigorously for 10 seconds. Repeat this step two more times. Then heat the vial in a water bath within a temperature range of 55-66°C for an additional seven minutes and shake vigorously. Use the doxorubicin HCl liposome injection within 8 hours. Refrigerate the vehicle and alkalinizing vials within a temperature range of 2-8°C until the time of use. The active agent is held at controlled room temperature within a range of 15-30°C.

### EXAMPLE 3

### TREATMENT

The effect of G-CSF on neutropenia as the dose limiting toxicity associated with administration of liposome encapsulated doxorubicin was observed. All 13 patients enrolled in the study had advanced malignancies, no prior therapy with anthracyclines and exhibited normal cardiac function and had no history of cardiac disease.

The composition of Example 1, doxorubicin HCL liposome injection, was administered intravenously with a minimum 21 g sized catheter over 1 hour to the patient on day one. Infusion was discontinued immediately if signs of irritation were noted at the site of administration. The initial dosage was 90 mg/m² for level 1 patients, 105 mg/m2 for level 2 patients, 120 mg/m2 for level 3 patients, 135 mg/m2 for level 4 patients and 150 mg/m2 for level 5 patients.

The subcutaneous administration of G-CSF was commenced on the fourth day following the administration of the liposome encapsulated doxorubicin, at an initial dosage of 5 mcg/kg/day, G-CSF, a recombinant human granulocyte colony stimulating factor produced by recombinant DNA technology in E. coli bacteria, is commercially available (Amgen, Inc., Thousand Oaks, Ca.). Vials of single-dose (300 mcg/ml of agent) are available preservative-free. The vials were refrigerated at 5°C. Prior to use, the drug was allowed to reach room temperature for a maximum of 6 hours.

The administration of G-CSF was continued until the patient's plasma absolute neutrophil count (ANC) is equal to or greater than 10.000/ul for 2 consecutive determinations after 14 days. The dosage of G-CSF was then tapered or de-escalated to 1 mcg/kg/day to avoid excessive leukocytosis and continued at this level until the plasma ANC was greater than 10,000/ul for 2 determinations. The administration of G-CSF was then discontinued. If at any time the patient's plasma ANC dropped to less than 2000/ul for more than 24 hours on the tapered dosage of G-CSF, the 5 mcg/kg/day dosage was reinstituted until the plasma ANC was greater than 10,000/ul for 2 consecutive determinations. The dosage was then re-tapered.

Treatment cycles were administered every 21 to 28 days. A second dosage of liposome encapsulated doxorubicin was administered not prior to 48 hours after the discontinuation of the G-CSF therapy, or the last treatment cycle.

Observations are indicated in Table 1.

**Table 1**

| Cycle 1 | | | | Cumulative Cycles | | | |
|---|---|---|---|---|---|---|---|
| dose level | no. of patients | ANC <500 | Platelets <20.000 | Total Cycles | ANC <500 | Platelets <20.000 | Neutro/fever |
| 1 | 3 | 1 | 0 | 11 | 2 | 1 | 1 |
| 2 | 3 | 1 | 1 | 14 | 5 | 4 | 2 |
| 3 | 4 | 2 | 2 | 10 | 5 | 6 | 3 |
| 4 | 3 | 2 | 1 | 4 | 2 | 2 | 2 |
| Total | 13 | 6 | 4 | 39 | 14 | 13 | 8 |

Cycle 1 was that cycle after the first injection with the liposome encapsulated doxorubicin. Cumulative cycles represents all cycles after Cycle 1.

Even though neutropenia was the most common toxicity, it was in most cases of a brief duration. Nonhematological toxicity was minor. Of the 39 treatment cycles only 3, one at dose level 1 and two at dose level 3, were associated with a Grade 3 mucositis, wherein severe oral ulcers were observed requiring a liquid diet. Three patients, two at dose level 2 and one at dose level 3, required dose reductions in later cumulative cycles. Nausea and vomiting, and fever associated with drug administration were well controlled in premedicated cycles. To date no cardiac toxicity has been observed in patients treated cumulatively with 400-786 mg/m2.

The composition of Example 2 was also employed in this Example.

## Claims

1. Use of a liposome encapsulated antineoplastic agent for the manufacture of a pharmaceutical composition for treating a neoplasm in combination with a hematopoietic cell stimulating agent, wherein the hematopoietic cell stimulating agent is not encapsulated in the liposome.

2. The use of claim 1 wherein the antineoplastic agent is vinblastine, etoposide, teniposide, vindesine, dactinomycin, doxorubicin, daunorubicin, mitomycin, methotrexate, 5-fluorouracil, cytarabine, 6-mercaptopurine, 5-azacytidine, hydroxyurea, 6-thioguanine, mechlorethamine, cyclophosphamide, melphalan, uracil mustard, chlorambucil, busulfan, carmustine, lomustine, semustine, carboplatin, thiotepa, ifosfamide, mesna, amsacrine, mitoxanthrone, or methotrexate with leucovorin, preferably wherein the antineoplastic agent is doxorubicin.

3. The used of claim 1 wherein the hamatopoietic cell stimulating agent is granulocyte-macrophage colony stimulating factor, granulocyte colony stimulating factor, or macrophage stimulating factor.

4. The use of claim 1 wherein the liposome comprises a chemical potential across its outer bilayer membrane, preferably wherein the chemical potential is a pH gradient.

5. The use of claim 1 wherein the liposome is comprised of a phospholipid.

6. The use of claim 3 wherein the granylocyte-macrophage colony stimulating factor, granulocyte colony stimulating factor, or macrophage stimulating factor is alternating with the use of the antineoplastic agent.

7. The use of claim 1 wherein the liposomes encapsulated antineoplastic agent dosage is escalated, preferably wherein the antineoplastic agent is doxorubicin.

8. The use of claim 1 wherein the maximum tolerated dose associated with the use of the liposome encapsulated antineoplastic agent to treat neoplasms in increased with the use of the hematopoietic cell stimulating agent so that myelosuppression is alleviated as a dose limiting toxicity.

9. Use of a liposome encapsulated antineoplastic agent for the manufacture of a pharmaceutical composition for alleviating myelosuppression as a dose limiting toxicity associated with the administration of liposome encapsulated antineoplastic agent in the treatment of a neoplasm comprising using an escalating dosage of an antineoplastic effective amount of liposome encapsulated antineoplastic agent and a hematopoietic cell stimulating effective amount of a hematopoietic cell stimulating agent, preferably wherein the antineoplastic agent is doxorubicin.

10. The use of claim 9 wherein the hematopoietic cell stimulating agent is a colony stimulating factor.
